# EUROPEAN PATENT SPECIFICATION

(11) **EP 0 509 968 B1**
(45) Date of publication and mention of the grant of the patent: **05.04.1995**
(21) Application number: 92830172.0
(22) Date of filing: 08.04.1992
(51) Int. Cl.: A61K 9/00, A61K 47/32, A61K 47/42

(54) **Synthetic polycarboxylates and proteins hydrogels for drug sustained release and process for the production thereof**
Synthetische Polycarboxylate und Proteine enthaltende Hydrogele als Träger von Wirkstoffen mit verzögerter Freisetzung und Verfahren zu ihrer Herstellung
Hydrogels contenant des polycarboxylates synthétiques et protéines pour médicaments à libération soutenue et leur procédé de production

(30) Priority: 15.04.1991 IT RM910260
(43) Date of publication of application: 21.10.1992
(73) Proprietor: ISTITUTO SIEROVACCINOGENO ITALIANO I.S.I. S.p.A., 55020 Castelvecchio Pascoli (Lucca) (IT)
(72) Inventor: Chiellini, Emo, I-56100 Pisa (IT); Solaro, Roberto, I-56100 Pisa (IT); Leonardi, Giuseppe, I-56100 Pisa (IT); Lisciani, Romero, I-00173 Rome (IT); Mazzanti, Giuseppe, I-55020 Castelvecchio Pascoli (Lucca) (IT)
(74) Representative: de Simone, Domenico

(56) References cited:
- EP-A- 0 179 477
- EP-A- 0 322 319
- JOURNAL OF POLYMER SCIENCE: POLYMER CHEMISTRY EDITION, vol. 22, no. 11, 1984,pages 2829-2839, John Wiley
- & Sons, Inc.; T. SUZUKI et al.: "Syntheses of monomethoxy polyethylenegylcolvinyl ether macromonomers and their radicalcopolymerization with maleic anhydride"

## Description

This invention relates to hydrogels based on synthetic polycarboxylates and proteins for the sustained release of drugs as well as to the process for the production thereof.

More particularly, this invention relates to hydrogels obtained through the combination of synthetic hydrocarbon-chain polymers containing carboxyl groups in side chains, with proteins or polypeptides, for the sustained release of drugs, for topical use, as well as to the process for the preparation thereof.

The advantages of matrices for the slow and sustained release of drugs for topical use to be administered through transdermal, ophthalmic, cutaneous routes etc. have been known for a long time, in all cases in which it is desirable not to subject the patient to many administrations and to keep the drug concentration constantly close to the minimum concentration capable of giving theurapetical effects.

Partly esterified copolymers based on maleic anhydride and alkyl vinyl esters have been investigated and suggested as new polymeric materials which can be employed as matrices for drug release owing to their dissolution properties.

Their general structure is mainly characterized by the presence of a carboxyl group which, becoming ionized at certain proper pH values, allows the polymeric chain to be solubilized. The dissolution capability can be remarkably modified by changing both the nature of the ester group adjacent to the carboxyl group, and the nature of the vinyl monomer substituent.

Derivatives of the polymeric material in question are reported in the technical literature in which derivatives the nature of the vinyl monomer substituent is a methyl group, and just the nature of the ester group is modified and investigated.

Various authors (Heller et al., J. Appl. Polym. Sci., 22, 1991-2009 (1978); A. Urtti et al., Ing. J. Pharm., 23, 147-161 (1985); Finne et al., J. Controlled Release, 10, 189-194 (1989)), have suggested as release means such polymeric matrices, which are made up of various hemiester derivatives of the alternate-structure copolymer of maleic anhydride and methyl vinyl ether, through the investigation of their employment for releasing different types of drugs which are not of protein nature.

Two of the present inventors (E.C. and G.L. jointly with other Authors) reported the preparation and employment of a series of hemiesters of alternate-structure copolymers of maleic anhydride with ethyl and butyl vinyl ether, for the formulation of ophthalmic inserts containing pilocarpine (Polymers in Medicine III - Biomedical and Pharmaceutical Applications, C. Migliaresi et al., Eds. Elsevier, Amsterdam 209, (1988)).

In that field, the following patent applications also to be reported:
- EP 0219207 relating to hydrophobic polymers which are polymerized by means of bonding agents, for drugs intended for transdermal topical use, more particularly for ophthalmic use. The formulations disclosed include hydrophobic polymers based on partially cross-linked polyacrylates and, in some cases, variable percentages of hydrophilic polymers. Polymers and copolymers (not specifically of alternate structure) based on maleic anhydride in percentages variable from 2 % to 15 %, and alkyl vinyl ethers, among which the butyl vinyl ether, have been inserted among the possible hydrophilic polymeric derivatives; polyalkylene glycols are employed for the formation of hydroxyalkyl esters of the acryl monomers;
- WO 89/06964 relating to microparticle suspensions or emulsions for ophthalmic employment, containing polymers based on acrylic acid and polyfunctional bonding agents with a low cross-linking degree, which rapidly gel in situ.
- EP 0082951 which relates to the preparation of active systems for sustained release of different principles (drugs, pesticides, perfumes, and so on), said systems consisting of variable composition hydrophilic copolymers based on maleic anhydride and polyalkylene glycol ethers wherein the active principle is covalently bonded to the polymer matrix. The release of the drug occurs owing to the slow hydrolysis of the bond between the active principle and the polymeric matrix.

The polymeric matrices already disclosed require remarkable amounts of glycerine or of ethylene glycol in order to ensure the characteristics of good formation of films and good plasticity which are suitable to the realization of inserts for the release of active principles. The diffusion loss of the plasticizer can indeed alter in time the main features of the insert itself so affecting in a not accurately definable way the release of the active principle.

Moreover, in the recent times protein nature compounds are increasingly employed for pharmacological treatments and investigations aiming to realize means for sustained release of such macromolecular drugs are becoming increasingly of interest.

In such context, the formulation of polymeric matrices having such requisites as to ensure a sustained release of the protein drug without damaging the intrinsic activity of the drug itself, takes a fundamental role in this new pharmaceutical practice, as is proved by recent meetings which are expressly devoted to such subject (2nd Int. Symp. on Disposition and Delivery of Peptide Drugs, Leiden, 1989).

Accordingly, the need for a hydrogel matrix turns out to be evident, in which matrix the hydrophilic/hydrophobic ratio can be controlled according to the compound to be delivered, so as to ensure a suitable solubility both in water and in water-organic or organic media, and which matrix is in combination with protein components and with active principles of polypeptide or protein nature and finally is easy to handle, so as to show better characteristics of film-formation and plasticity, said characteristics being connected to lower values of glass transition, without requiring the employment of additives like glycerine or ethylene glycol.

The Authors of the present invention have found that hydrogels comprising a synthetic polymeric matrix based on maleic anhydride and polyoxyethylene glycol monomethyl ethers, vinyl ethers, and a protein component give, following the process that will be disclosed below, uniform and transparent laminae with water solubility better than the values reported up to the present time and with adjustable hydrophilic/hydrophobic ratios. This seems to be the result of the different structure of the alkyl group which is present on the vinyl monomer. Moreover, the hydrophilic component of the polymer is covalently anchored to the main chain through a bond of the ether type, with improved properties of structural integrity against physicochemical agents with respect to polymers in which the hydrophilic component is bonded to the polymeric matrix through an ester-type bond.

The hydrogel matrices so formed are useful for the sustained release of drugs, in particular for drugs of polypeptide or protein nature, which are tolerated by patients in a very good way and can be employed also for inserts for ophthalmic use. The possibility of adjusting the hydrophilic/hydrophobic balance makes finally such matrices very suitable for adaptation to the release of different active principles. The protein component which is not pharmacologically active makes the hydrogel formation easier, with erosion features capable of being adjusted according to structural bases.

Moreover, the object of this invention is also the supplying of a process for the preparation of said hydrogel matrices, said process comprising the formation of polymers through the addition of maleic anhydride to alkyl vinyl ethers obtained from oligoxyethylene and oligoxypropylene monoalkylates selectively and successive partial esterification on the anhydride residues level, with alcohols.

The hydrophilic/hydrophobic balance of the systems pointed out above is controlled on structural and composition bases, so as to ensure a suitable solubility in water and in water-organic and organic media.

By addition of the protein component to the various kinds of hemiesters followed by a controlled evaporation, first at a low temperature and then at 50°C, it is possible to obtain gel matrices in the form of a variable thickness film, from which film inserts can be cut out which swell uniformly when dipped in water and under physiological conditions up to volumes which are variable between 5 and 20 times the initial volume. The swollen inserts undergo an erosive dissolution process in the conditions pointed out above, with a weight loss gradient which is constant in time. This type of behaviour together with the coherence of shape of the insert during the surface erosion process make useful biocompatible supports of such formulations, said supports having optimal perspective employments in the sustained release of the active principle with a constant gradient. More particularly, in the case of inserts containing interferon as the active principle, it has been possible to estimate the time for the total delivery of the whole dose employed in 1-4 weeks.

Moreover, starting from such systems, through the addition of disgregating agents of the type of linear and cross-linked polyvinyl pyrrolidone, polyvinyl alcohol, dextrans and modified dextrins, it is possible to realize systems showing immediate release of active protein principles, while theier cross-linking through chemical means and through physical means might lead to the realization of systems showing a more prolonged sustained release.

Accordingly, it is the object of this invention a hydrogel matrix for the sustained release of pharmacologically active compounds, said hydrogel matrix comprising a synthetic polymer having a hydrocarbon chain of the formula: 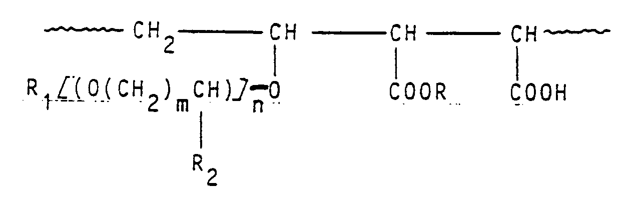
wherein R = (C₁-C₁₂) alkyl; R₁ = (C₁-C₄) alkyl, R₂ = H (m = 1,3; n = 1-12) or CH₃ (m = 1; n = 1-12); a protein component and a pharmacologically active principle.

Again according to this invention, said hydrocarbon chain synthetic polymer is derived by the polymerization of maleic anhydride with a monoalkyloxyoligoethylene glycol vinyl ether, preferably included in the group of 2-methoxyethyl vinyl ether, monomethoxytriethylene glycol vinyl ether, monomethoxytetraethylene glycol vinyl ether and by the successive hemiesterification through the reaction with alcohols, with a variable hydrophilic/hydrophobic balance.

Advantageously, said protein component is an albumin, and preferably it is the human seric albumin (HSA).

According to the present invention, the weight ratio of the protein component to the polymer is comprised in the range from 0.01 and 1, and preferably is between 0.1 and 0.5.

Again according to this invetion, said pharmacologically active principle comprises a protein or a polypeptide, preferably an interferon, and even more preferably alpha-interferon.

The hydrogel matrix which is the object of this invention can be applied for sustained release of said compounds showing pharmacological activity through the transcutaneous, the transdermal and ophthalmic routes, but preferably for ophthalmic use.

It is also an object of the present invention a process for the preparation of said hydrogel matrices, said process comprising the steps of: preparing said synthetic polymers through polymerization; their conversion into the corresponding hemiesters by reaction with alcohols; addition of said protein component to a solution of said polymer; controlled evaporation.

According to said process, said polymers are prepared by polymerization of maleic anhydride with monoalkyloxyoligoethylene glycol vinyl ethers, preferably comprised in the group of 2-methoxyethyl vinyl ether, monomethoxytriethylene glycol vinyl ether and monomethoxytetraethylene glycol vinyl ether, and said hemiesterification occurs by reaction with alcohols, preferably comprised in the group of methanol, n-butanol and lauryl alcohol.

Again according to this invention said protein component is added to a solution of said polymer in organic solvent, or in water-organic or water-containing media as solvents, in which said polymer is present in weight percentages between 1 % and 50 %, and in which the weight ratios of the protein component to the polymers are in the range from 0.01 and 1, preferably between 0.1 and 0.5.

The controlled evaporation occurs at a temperature between -70°C and 0°C and next between 0°C and 50°C, till formation of films, which are preferably of thickness between 10 »m and 500 »m.

Again according to this invention, it is possible to add a cross-linking agent of divinyl nature, preferably selected from among methylenebisacrylamide, divinylbenzene, ethylene glycol dimethacrylate, diethylene glycol divinyl ether, in amounts between 0.2 % and 20 % by weight, preferably between 2 % and 5 %.

Alternatively, said cross-linking agent can be of epoxidic, isocyanic or amino nature.

This invention will be disclosed in the following with reference to some preferred embodiments of the same, which are illustrated in the following examples, wherein the examples 1-4 disclose the preparation of some monomers; examples 5-7 disclose the preparation of copolymers of maleic anhydride/alkyl vinyl ethers; examples 8-13 disclose the hemiesterification procedure of said copolymers, while examples 14-14 disclose the preparation of hydrogels; examples 16-17 disclose hydration tests of hydrogels; and example 18 discloses a drug release test in vitro.

The following figures will be referred to in said examples, in which figures:
- Figure 1 illustrates the scheme of a typical apparatus for the production of hydrogels containing proteins according the present invention;
- Figure 2 shows a diagram of the weight loss and hydration of a specific hydrogel matrix according to this invention, as a function of time;
   Figure 3 shows a diagram of the release of interferon from a matrix according to the present invention.

### Example 1

### Preparation of 2-methoxyethyl vinyl ether (Peg IVE)

### a) 2-Methoxyethanol

96.5 g (1.27 moles) of 2-methoxyethanol (Peg 1Me) and 25 ml of benzene are put into a three-neck flask of 500 ml volume, provided with a bubble condenser, a dropping funnel and a magnetic stirrer, under a nitrogen blanket. The mixture was dried by azeotropic distillation and successive removal of excess benzene, and next the mixture was cooled to 0°C. Then 4.0 g (0.166 moles) of NaH suspended in 60 ml of n-hexane was added slowly to the mixture. After finishing the addition, the mixture was heated up to 90°C during 2 hours and then, after cooling the same down to room temperature, 23.6 g (0.166 moles) of methyl iodide was added. The mixture was heated up to 80°C for 3 hours and then it was cooled to room temperature. After removing n-hexane under atmospheric pressure, the mixture was distilled under reduced pressure, and the fraction with boiling point 34-36°C/20 mm (yield 74 %) was collected.

### b) Vinylation of 2-methoxyethanol

57.0 g (0.75 moles) of Peg 1Me and, in small portions, 3.0 g (77 mmoles) of metallic potassium previously washed with n-hexane were put into a two-neck, 250 ml flask provided with a bubble condenser and a mechanical stirrer, under a nitrogen blanket. When the addition was over, and after completion of the formation of the alcoholate for 1 hour, the solution was transferred into an autoclave of 250 ml volume provided with a magnetic stirre and, after evacuation, acetylene was introduced up to 12 atmosphere pressure. The mixture was stirred and heated up to 100°C for 8 hours and then it was cooled down to room temperature. The loading operation was repeated 9 times. The product, recovered from the autoclave by means of ethyl ether, after removal of ether under atmospheric pressure, was distilled under reduced pressure, so collecting a single fraction with boiling point 20-22°C/20 mm.

The product was refluxed over Na-K alloy for 3 hours and then it was distilled under reduced pressure, and the fraction with boiling point 20-22°C/20 mm was collected (50 % yield).

The product so obtained (Peg 1VE) has the following formula: 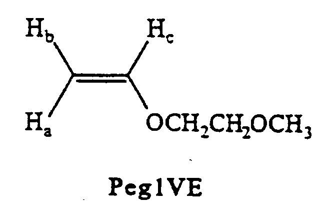
IR (liquid film) ν̅ = 3120-3020 (ν vinyl CH), 3000-2820 (ν aliphatic CH), 1618 (ν C=C), 1456 (δ CH₂ and δₐ CH₃), 1380 (δₛ CH₃), 1204 (νₐ C-O-C=), 1130 (ν C-O-C), 1096 (νₛ C-O-C=), 968 and 818 cm⁻¹ (δ vinyl CH).

¹H-NMR (CDCl₃) δ = 6.5 (dd, 1H, =CH_{c}), 4.2 (dd, 1H, =CHₐ), 4.0 (add, 1H, =CH_{b}), 3.8 and 3.6 (two m, 4H, CH₂) and 3.4 ppm (s, 3H, CH₃).

### Example 2

### Preparation of triethylene glycol monomethyl ether vinyl ether (Peg3VE)

### a) Triethylene glycol monomethyl ether (Peg3Me)

191.2 g (1.27 moles) of Peg3Me and 50 ml of benzene were introduced into a three-neck, 1 l flask provided with a bubble condenser, a magnetic stirrer, and a dropping funnel, under a nitrogen blanket. The mixture was dried by azeotropic distillation and successive removal of excess benzene, and next the mixutre was cooled down to 0°C. Then 7.6 g (0.318 moles) of NaH suspended in 80 ml of n-hexane was slowly added to the mixture. After finishing the addition, the mixture was heated up to 90°C for 2 hours, and then after cooling the same to room temperature, 45.2 g (0.318 moles) of methyl iodide was added. The mixture was then heated up to 80°C for 3 hours and then it was cooled down to room temperature. After removing n-hexane under atmospheric pressure, the mixture was distilled under reduced pressure collecting the fraction having boiling point of 80-82°C/0.05 mm.

### b) Vinylation of triethylene glycol monomethylether

121 g (0.737 moles) of Peg3Me and, in small portions. 2.88 g (0.737 moles) of metallic potassium previously washed with n-hexane were introduced into a three-neck, 500 ml flask provided with a bubble condenser and a magnetic stirrer under anhydrous nitrogen blanket. When the addition was finished and after completion of formation of the alcoholate during one hour, the solution was transferred into a 250 ml autoclave provided with a magnetic stirrer, and after evacuation of the same, acetylene was introduced up to 12 atmospheres pressure. The loading operation was repeated eleven times. The product recovered from the autoclave by ethyl ether was put into a 250 ml Claisen flask and then distilled, first under atmospheric pressure for removing ethyl ether and next under reduced pressure, and the fraction having boiling point of 60-63°C/0.1 mm was collected. The product was refluxed over sodium for 5 hours and it was distilled under reduced pressure, and the fraction having boiling point of 60-62°C/0.1 mm was collected (54 % yield).

The product so obtained (Peg3VE) has the following formula: 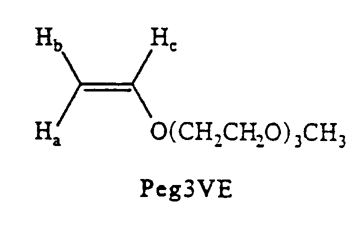
IR (liquid film) ν̅ = 3120-3020 (ν vinyl CH), 3000-2800 (ν aliphatic CH), 1618 (ν C=C), 1456 (δ CH₂ and δₐ CH₃), 1380 (δₛ CH₃), 1202 (νₐ C-O-C=), 1112 (ν C-O-C), 1034 (νₛ C-O-C=), 982 and 820 cm⁻¹ (δ vinyl CH).
¹H-NMR (CDCl₃) δ = 6,5 (dd, 1H, =CH_{c}), 4,2 (dd, 1H, =CHₐ), 4,0 (dd, 1H, =CH_{b}), 3,8-3,5 (m, 12H, CH₂), 3,4 ppm (s, 3H, CH₃).

### Example 3

### Preparation of tetraethylene glycol monomethylether vinyl ether (Peg4VE)

### a) Tetraethylene glycol monomethylether (Peg4Me)

112.4 g (0.54 moles) of tetraethylene glycol and 200 ml of dioxane were introduced under nitrogen blanket into a three-neck 1 l flask provided with bubble condenser, magnetic stirrer and dropping funnel. Then 19.3 g (0.803 moles) of NaH suspended in 200 ml of dioxane was added slowly. The mixture was then kept stirred for 10 hours at room temperature and then 114 g (0.803 moles) of methyl iodide in 200 ml of dioxane was slowly added to the same. The mixture was kept under reflux for 20 hours. After filtering, dioxane was removed under vacuum. The product was not purified further.

### b) Vinylation of tetraethylene glycol monomethylether

70 g (0.336 moles) of Peg4Me and in small portions, 1.32 g (0.0336 moles) of metallic potassium previously washed with n-hexane were introduced under anhydrous nitrogen blanket into a three-neck, 500 ml flask provided with a bubble condenser and a magnetic stirrer. When the addition was finished and after completion of formation of the alcoholate for 1 hour, the solution was transferred into a 250 ml autoclave provided with a magnetic stirrer, and after evacuation of the autoclave, acetylene was introduced up to 12 atmospheres pressure. The loading operation was repeated 5 times. The product recovered from the autoclave by means of ethyl ether was distilled, first uder atmospheric pressure for removing ethyl ether and next under reduced pressure, and the fraction having boiling point of 102-105°C/0.05 mm was collected. The product was distilled over sodium under reduced pressure, and the fraction having boiling point of 102-105°C/0.05 mm was collected (48 % yield).

The product so obtained (Peg4VE) has the following formula: 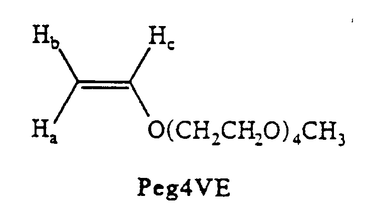
IR (liquid film) ν̅ = 3116-3020 (ν vinyl CH), 3000-2800 (ν aliphatic CH), 1618 (ν C=C), 1456 (δ CH₂ and δₐ CH₃), 1380 (δₛ CH₃), 1202 (νₐ C-O-C=), 1112 (ν C-O-C), 1038 (νₛ C-O-C=), 982 and 848 cm⁻¹ (δ vinyl CH).
¹H-NMR (CDCl₃) δ = 6,5 (dd, 1H, =CH_{c}), 4,2 (dd, 1H, =CHₐ), 4,0 (dd, 1H, =CH_{b}), 3,8-3,5 (m, 16H, CH₂) , 3,4 ppm (s, 3H, CH₃).

### Example 4

### Purification of maleic anhydride (AnM)

The product commercially available was recrystallized from benzene under nitrogen blanket before employment, taking care of separating maleic acid possibly present by hot filtering the benzene solution. The recrystallized product had melting point of 54-56°C.

Copolymerization tests of various alkyl vinyl ethers with maleic anhydride were carried out following the same procedure. Some typical experiments are disclosed below in detail.

### Example 5

### Copolymerization of 2-methoxyethyl vinyl ether with maleic anhydride (AnM/Peg1VE)

450 ml of benzene, 29.4 g (0.3 moles) of AnM and, with stirring, 30.6 g (0.3 moles) of Peg1VE and 0.32 g (0.02 moles) of AIBN were introduced under nitrogen blanket into a 1 l flask provided with a bubble condenser and a magnetic stirrer. After degassing, the mixture was kept stirred at room temperature for 15 hours and next it was heated up to 60°C for 15 hours. The contents of the flask cooled down to room temperature were poured slowly into anhydrous ethyl ether with vigorous stirring. The polymer so precipitated was filtered under anhydrous nitrogen blanket, then it was purified through reprecipitation from acetone in ethyl ether and then it was dried under vacuum till constant weight. 43.5 g of the product (72 % yield) was thus obtained.

IR (liquid film) ν̅ = 3000-2900 (ν aliohatic CH), 1856-1780 (ν C=O), 1454 (δ CH₂ and δₐ CH₃); 1378 (δₛ CH₃), 1224 and 1104 cm⁻¹ (ν C-O-C).
¹H-NMR (CDCl₃) δ = 4,7-3,4 (m, 5H, CH-O + CH₂O), 3,3 (m, 3H, OCH₃) , 2,7-1,9 ppm (s, 4H, CH-COO + CH₂).

### Example 6

### Copolymerization of monomethoxytrietylene glycol vinyl ether with maleic anhydride (AnM/Peg3VE)

600 ml of benzene, 24.5 g (0.25 moles) of AnM and then, with stirring, a mixture containing 86 % Peg3VE (0.25 moles) and 14 % Peg3-dimethylether (0.25 moles) and 0.27 g (1.66 mmoles) of AIBN were introduced under nitrogen blanket into a 1 l flask provided with a bubble condenser and a magnetic stirrer. The mixture was frozen twice with liquid nitrogen, then degassed under the mechanical pump vacuum and then it was slowly heated up to room temperature. The solution was then kept stirred at room temperature for 15 hours and then it was heated up to 60°C for 45 hours. The contents of the flask, after cooling the same down to room temperature, were slowly poured into anhydrous ethyl ether with vigorous stirring. The polymer so obtained was purified by reprecipitation from acetone in a large excess of ethyl ether and then it was dried under vacuum till constant weight. 58.0 g of the product was thus obtained (81 % yield).
IR (liquid film) ν̅ = 3000-2800 (ν aliphatic CH), 1856-1772 (ν C=O), 1456 (δ CH₂ and CH₃), 1378 (δₛ CH₃), 1224 and 1104 cm⁻¹ (ν C-O-C)
¹H-NMR (CDCl₃) δ = 4,7-3,4 (m, 5H, CH-O + CH₂O), 3,3 (m, 3H, OCH₃) , 2,7-1,9 ppm (s, 4H, CH-COO + CH₂).

### Example 7

### Copolymerization of monomethoxytetraethylene glycol vinyl ether with maleic anhydride (AnM/Peg4VE)

37 g of a mixture containing 10 % Peg4VE (16 mmoles) and 90 % Peg3-dimethylether, and then, with stirring, 1.6 (16 mmoles) of AnM and 17 mg (0.15 mmoles) of AIBN were introduced under nitrogen blanket into a 100 ml flask provided with a bubble condenser and a magnetic stirrer. The mixture was then frozen twice with liquid nitrogen, then it was degassed by mechanical pump vacuum and the mixture was then slowly heated up to room temperature. The solution was kept stirred at room temperature for 20 hours and then it was heated up to 60°C for 30 hours. The contents of the flask, which was cooled down to room temperature, were then poured slowly into anhydrous ethil ether with vigorous stirring. The polymer so obtained was then purified through reprecipitation from acetone in a large excess of ethyl ether and then it was dried under vacuum till constant weight. 1-2 g of the product was thus obtained (40 % yield).
IR (liquid film) ν̅ = 3000-2800 (ν aliphatic CH), 1856-1772 (ν C=O), 1456 (δ CH₂ and δₐ CH₃), 1380 (δₛ CH₃), 1224 and 1104 cm⁻¹ (ν C-O-C).
¹H-NMR (CDCl₃) δ = 4,7-3,4 (m, 5H, CH-O + CH₂O), 3,3 (m, 3H, OCH₃) , 2,7-1,9 ppm (s, 4H, CH-COO + CH₂).

The hemiesterification reactions with different alcohols of the copolymerization products of maleic anhydride with monomethoxyoligoethylene glycol vinyl ethers were carried out following usual procedures. The experimental details of some typical examples are reported in the following.

### Example 8

### Hemiesterification of the AnM/Peg1VE copolymer with methanol

80 ml of tetrahydrofuran and 10.0 g (0.05 moles of AnM units) of the AnM/Peg1VE copolymer were introduced under nitrogen blanket into a three-neck, 250 ml flask provided with a bubble condenser, dropping funnel and mechanical stirrer, and the mixture was stirred while hot till complete dissolution of the copolymer. 19.7 g (0.615 moles) of methanol was added dropwise to such solution. When the addition was finished, the solution was refluxed for 86 hours, then it was cooled down to room temperature and poured slowly with vigorous stirring into excess ethyl ether, then it was collected and dried under vacuum till constant weight. 9.8 g of the product were thus obtained.
IR (liquid film) ν̅ = 3500-2500 (ν CH and ν acid OH), 1734 (ν C=O), 1460 (δ CH₂ and δₐ CH₃), 1380 (δₛ CH₃, 1250-1000 cm⁻¹ (ν C-O-C).
¹H-NMR (DMSO-d₆) δ = 12,2 (s, 1H, COOH), 3,6 (s, 3H, CH₃OOC), 3,4 (s, 5H, CH₂O + CHO), 3,3 (m, 3H, OCH₃), 2,7 (s, 2H, CH-COO), 1,9 ppm (s, 2H, CH₂).

### Example 9

### Hemiesterification of the AnM/pegIVE copolymer with n-butanol

100 ml of tetrahydrofuran and 10.0 g (0.05 moles of AnM units) of the AnM/Peg1VE copolymer were introduced under nitrogen blanket into a three-neck, 250 ml flask, provided with a bubble condenser, dropping funnel and mechanical stirrer, and the mixture was stirred while hot till full dissolution of the copolymer. 40.5 g (0.546 moles) of n-butanol was then added to such solution. After finishing the addition, the solution was refluxed for 192 hours, then it was cooled down to room temperature and poured slowly into ethyl ether with vigorous stirring. The product so obtained was purified through successive repeated precipitations from acetone in a large excess of ethyl ether, then it was collected and dried under vacuum till constant weight. 13.5 g of the product were thus obtained.
IR (liquid film) ν̅ = 3500-2500 (ν CH and ν acid OH), 1734 (ν C=O), 1460 (δ CH₂ and δₐ CH₃), 1380 (δₛ CH₃), 1250-1000 cm⁻¹ (ν C-O-C).
¹H-NMR (DMSO-d₆) δ = 12,2 (s, 1H, COOH), 4,0 (s, 2H, CH₂OOC), 3,4 (s, 5H, CH₂O + CHO), 3,3 (m, 3H, OCH₃), 2,7 (s, 2H, CH-COO), 2,1-1,0 (m, 6H, CH₂), 0,9 ppm (s, 3H, CH₃).

### Example 10

### Hemiesterification of the AnM/Peg1VE copolymer with lauryl alcohol

100 ml of tetrahydrofuran and 8.0 g (0.04 moles of AnM units) of the AnM/Peg1VE copolymer were introduced under nitrogen blanket into a three-neck, 250 ml flask provided with a bubble condenser, dropping funnel and mechanical stirrer, and the mixture was then stirred while hot till full dissolution of the copolymer. A solution of 89.4 g (0.48 moles) of lauryl alcohol in 80 ml of tetrahydrofuran was added to the clear solution. When the addition was finished, the solution was refluxed for 245 hours, then it was cooled to room temperature and next it was transferred, in small portions, into a separating funnel where it was treated with a 10 % sodium bicarbonate solution. The white gel-like precipitate so formed, after agitation with ethyl ether both to favour its agglomeration and to solubilize the excess lauryl alcohol, was filtered and then suspended for some hours, with vigorous stirring, in ethyl ether and next in acetone. After separating acetone through decantation, the product was dried under vacuum till constant weight. 9.5 g of the polymer product was recovered in the form of the sodium salt.
IR (liquid film) ν̅ = 3500-2500 (ν CH and ν acid OH), 1734 (ν C=O), 1460 (δ CH₂ and δₐ CH₃), 1380 (δₛ CH₃), 1250-1000 cm⁻¹ (ν C-O-C)
¹H-NMR (DMSO-d₆) δ = 12,2 (s, 1H, COOH), 4,0 (s, 2H, CH₂OOC), 3,4(s, 5H, CH₂O + CHO), 3,3 (m, 3H, OCH₃), 2,7 (s, 2H, CH-COO), 2,1-1,0 (m, 22H, CH₂), 0,9 ppm (s, 3H, CH₃).

### Example 11

### Hemiesterification of the AnM/Peg3VE copolymer with methanol

100 ml of tetrahydrofuran and 10.0 g (35 mmoles of AnM) of the AnM/Peg3VE copolymer were introduced under nitrogen blanket into a three-neck, 250 ml flask provided with a bubble condenser, dropping funnel and mechanical stirrer, and the mixture was stirred while hot till complete dissolution of the copolymer. 27.7 g (0.863 moles) of methanol was added dropwise to the clear solution. When the addition was finished, the solution was refluxed for 136 hours, then it was cooled down to room temperature and precipitated in n-hexane with vigorous stirring. The product so obtained was purified through successive reprecipitations from acetone in a large excess of hexane, the it was collected and dried under vacuum till constant weight. 7.5 g of the product were thus obtained.
IR (liquid film) ν̅ = 3500-2500 (ν CH and ν acid OH), 1734 (ν C=O), 1460 (δ CH₂ and δₐ CH₃), 1380 (δₛ CH₃), 1250-1000 cm⁻¹ (ν C-O-C) ¹H-NMR (DMSO-d₆) δ = 12,2 (s, 1H, COOH), 3,6 (s, 3H, CH₃OOC), 3,4 (s, 13H, CH₂O + CHO), 3,3 (m, 3H, OCH₃), 2,7 (s, 2H, CH-COO), 1,9 ppm (s, 2H, CH₂).

### Example 12

### Hemiesterification of the AnM/Peg3VE copolymer with n-butanol

100 ml of tetrahydrofuran and 10.0 g (35 mmoles of AnM units) of the AnM/Peg3VE copolymer were introduced into a three-neck, 250 ml flask provided with a bubble condenser, dropping funnel and mechanical stirrer, under nitrogen blanket, and the mixture was stirred while hot till complete dissolution of the copolymer. 28.4 g (0.382 moles) of n-butano was added dropwise to the clear solution. When the addition was finished, the solution was refluxed for 224 hours, then it was cooled down to room temperature and poured slowly into n-hexane with vigorous stirring. The product so obtained was purified by means of successive reprecipitation from acetone in a large excess of n-hexane, then the product was collected and vacuum dried till constant weight. 8.5 g of the product was so obtained.
IR (liquid film) ν̅ = 3500-2500 (ν CH and ν acid OH), 1734 (ν C=O), 1460 (δ CH₂ and δₐ CH₃), 1380 (δₛ CH₃), 1250-1000 cm⁻¹ (ν C-O-C).
¹H-NMR (DMSO-d₆) δ = 12,2 (s, 1H, COOH), 4,0 (s, 2H, CH₂OOC), 3,4 (s, 13H, CH₂O + CHO), 3,3 (m, 3H, OCH₃), 2,7 (s, 2H, CH-COO), 2,1-1,0 (m, 6H, CH₂), 0,9 ppm (s, 3H, CH₃).

### Example 13

### Hemiesterification of the AnM/Peg3VE copolymer with lauryl alcohol

100 ml of tetrahydrofuran and 7.2 g (25 mmoles of AnM units) of the AnM/Peg3VE copolymer were introduced under nitrogen blanket into a three-neck, 250 ml flask provided with a bubble condenser, dropping funnel and mechanical stirrer, and the mixture was stirred while hot till complete dissolution of the copolymer. A solution of 56.0 g (0.3 moles) of lauryl alcohol in 50 ml of tetrahydrofuran was added to the clear solution. When the addition was finished, the solution was refluxed for 245 hours, then it was cooled down to room temperature and next transferred in small portions into a separating funnel where it was treated with a 10 % sodium bicarbonate solution. The quite gel-like precipitate after washing with n-hexane and next with acetone, was suspended in ethyl ether with vigorous stirring for some hours. After decantation of ethyl ether, the polymer was vacuum dried till constant weight. 9.2 g of the polymeric product was recovered in the form of the sodium salt.
IR (liquid film) ν̅ = 3500-2500 (ν CH and ν acid OH), 1734 (ν C=O), 1460 (δ CH₂ and δₐ CH₃), 1380 (δₛ CH₃), 1250-1000 cm⁻¹ (ν C-O-C).
¹H-NMR (DMSO-d₆) δ = 12,2 (s, 1H, COOH), 4,0 (s, 2H, CH₂OOC), 3,4 (s, 13H, CH₂O + CHO), 3,3 (m, 3H, OCH₃), 2,7 (s, 2H, CH-COO), 2,1-1,0 (m, 22H, CH₂), 0,9 ppm (s, 3H, CH₃).

### Example 14

### Preparation of a hydrogel based on the methyl hemiester of the AnM/Peg1VE copolymer and human seric albumin (HSA) containig alfa-interferon (IFNₐₗₚₕₐ)

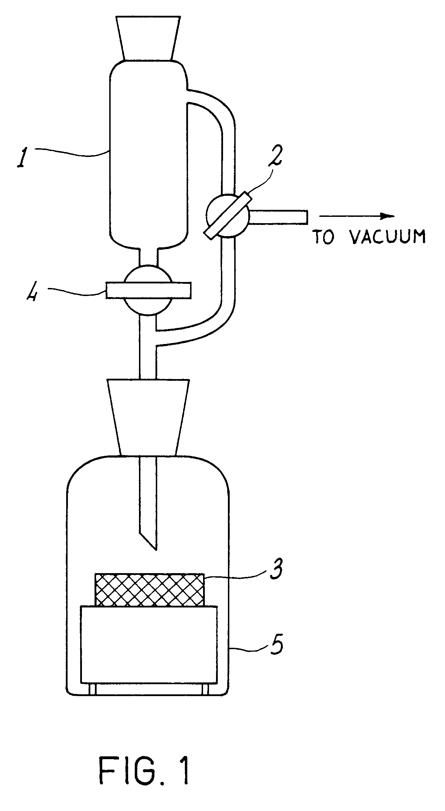

A Teflon dish 3 of 3 cm diameter was put inside a ground-glass neck bottle 5 provided with a dropping funnel 1 of 10 ml volume and endowed with a three-way cock 2 (see Figure 1) and a pressure-equilibration chamber, containing a small magnetic anchor after removing dampness through a stream of anhydrous nitrogen, the apparatus was put inside a Dewar bottle and cooled down to -50°C by means of dry ice (solid CO₂) Then a solution of 300 mg of methyl hemiester of the AnM/Peg1VE copolymer in 2 ml of acetone and containing 25 »l of glycerine as a plasticizer, and cooled to -20°C, was put into the dropping funnel 1. A protein suspension in acetone, prepared as follows, was then added to such solution. A solution of 30 mg of a freeze-dried product based on human seric albumin, containing 120,000 I.U. of IFN_{beta} in 1 ml of distilled water was then added dropwise into a centrifuge test-tube containing 6 ml of acetone cooled down to -20°C; the precipitate so obtained was rapidly centrifuged (1,500 gpm, 2 minutes) and then it was resuspended in 1 ml of acetone at -20°C. The resulting suspension was then stirred magnetically for 30 seconds and then introduced dropwise and rapidly into the dish 3 by means of the cock 4. When the inside temperature of the apparatus became stabilized at -60°C, the solvent was partially removed under vacuum (300-20 mm Hg) for 6 hours.

The temperature was then allowed to rise slowly up to the room temperature value, reducing proportionally the applied vacuum in order to prevent bubbles from forming inside the film, and the drying operation was carried out in a dry air stream for four hours. Next the dish was transferred into a oven at 45°C for 24 hours. Thus a homogeneous and flexible (and slightly opaque) film of 250 »m thickness was obtained, from which some circular inserts of 3 mm diameter were obtained through cutting by a suitable punch.

### Example 15

### Preparation of a hydrogel based on the methyl hemiester of the AnM/Peg3VE copolymer containing HSA + IFNₐₗₚₕₐ

A solution of 300 mg of the methyl hemiester of the AnM/Peg3VE copolymer in 2 ml of acetone cooled down to -20°C was put into the dropping funnel of the apparatus shown in Figure 1, kept at -50°C. A suspension of protein in acetone was added to such solution, the suspension being prepared as follows. A solution of 30 mg of a freeze-dried product based on human seric albumin (HSA) containing 120,000 I.U. of IFN_{α} in 1 ml of distilled water was added dropwise to a centrifuge test-tube containing 6 ml od acetone cooled to -20°C; the precipitate so obtained was rapidly centrifuged (1500 rpm, 2 minutes) and then resuspended in 1 ml of acetone at -20°C. The resulting suspension was then stirred magnetically for 30 seconds and then added dropwise rapidly to the dish. When the inside temperature of the apparatus became stabilized at -60°C, the solvent was partially removed under vaccum (300-20 mm Hg) for 6 hours. Then the temperature was allowed to come back to the room temperature value slowly, while proportionally reducing the applied vacuum in order to prevent bubbles from forming inside film, and the drying operation was carried out after that in a dry air stream for 4 hours. Next the dish was transferred into an oven at 45°C for 24 hours. Thus a homogeneous and flexible film was obtained (which was slightly opaque) of 300 »m thickness, from which a number of circular inserts of 3 mm diameter were obtained by cutting with a suitable punch.

### EXAMPLES OF HYDRATION TESTS OF THE INSERTS

Water absorption tests (hydration tests) were carried out by putting the inserts into a stainless steel net bucket (22 mm diameter, 24 mm height) divided into three sectors in order to have the possibility of analyzing three inserts simultaneously. The bucket dipped in 40 ml of a 1.33 mM phosphte buffer solution at pH 7.4, was caused to rotate at a constant speed of 60 rpm and at a temperature of 37°C. The inserts were removed at intervals from the water solution, then dried superficially and weighed; then the samples were put into an oven kept at 45°C, and they were allowed to dry for 24 hours, and afterward they were weighed again.

### Example 16

Three polymeric inserts based on n-butyl hemiester of the Peg1VE/AnM copolymer containing HSA + IFNₐₗₚₕₐ of 2.63 mg ± 0.20 mg weight (3 mm diameter and 360 »m + 15 »m thickness) were put into a stainless steel net bucket divided into three sectors. The bucket was then dipped into 40 ml of a 1.33 mM isotonic phosphate buffer solution at pH 7.4, said solution being kept at a temperature of 37°C ± 0.1°C and then the bucket was caused to rotate at a constant speed of 60 rpm.

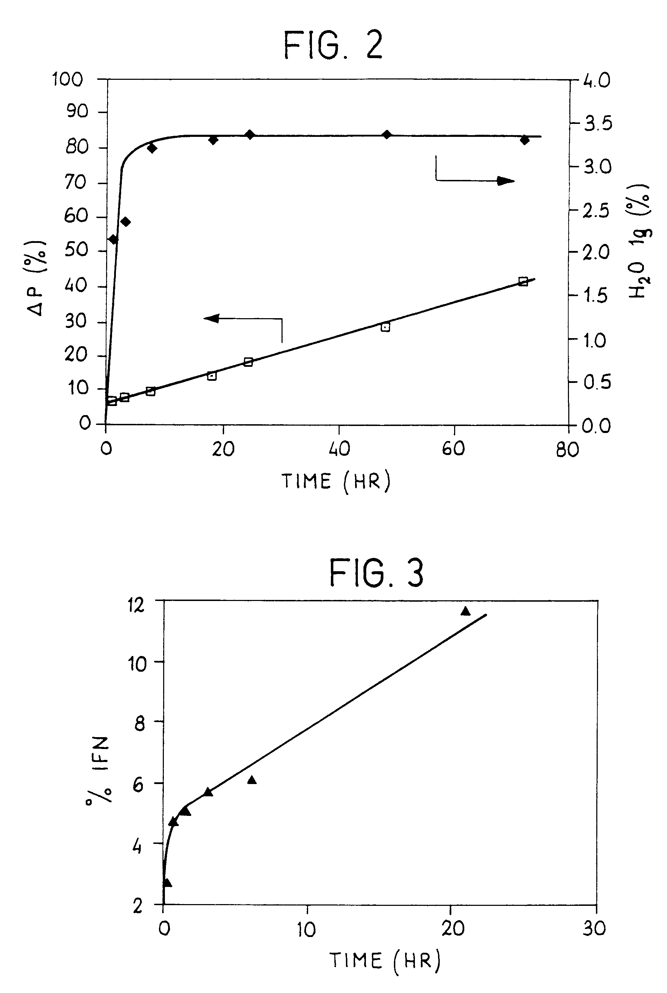

At proper time intervals the bucket was removed from the solution and the inserts were drawn from the bucket, and then they were superficially dried and weighed; the inserts were then put into an oven kept at 45°C, and were allowed to dry for 24 hours and then they were weighed again. Percentage weight losses and the absorbed water percentage at the various times, calculated with respect to the initial dry weight, are summarized in Table 1. Figure 2 illustrates the time behaviour of hydration percentages and weight losses.

**Table 1**

| Hydration and weight loss tests of polymeric inserts based on n-butyl hemiester of the Peg1VE/AnM copolymer and containing HSA + IFNₐₗₚₕₐ | | |
|---|---|---|
| Time hours | weight loss % | hydration ^{a)} % |
| 0.25 | 6.6 | 140 |
| 3 | 8.1 | 230 |
| 7.5 | 9.7 | 1640 |
| 18 | 14.4 | 2000 |
| 24 | 18.5 | 2280 |
| 48 | 28.6 | 2250 |
| 72 | 41.8 | 1930 |

| | | |
|---|---|---|
| a) Average percentage values calculated over three inserts, the values being referred to the dry weight of the sample. | | |

### Example 17

Three polymeric inserts based on methyl hemiester of the PegIVE/AnM copolymer containing HSA + IFNₐₗₚₕₐ of 2.34 mg ± 0.20 mg weight (3 mm diameter and 320 »m ± 15 »m thickness) were put into a stainless steel net bucket divided into three settors. The bucket was then dipped into 40 ml of an isotonic 1.33 mM phosphate buffer solution at pH 7.4, kept at a temperature of 37°C ± 0.1°C, and finally the bucket was put into constant speed rotation at 60 rpm. At proper time intervals the bucket was drawn out the solution and the inserts were then carefully drawn out of the same, then they were dried superficially and weighed; the inserts were then put into an oven at 45°C and allowed to dry for 24 hours and then they were weighed again. The percentage weight losses and the percentage of absorbed water at the various times, calculated with respect to the dry weight, are summarized in Table 2.

**Table 2**

| Hydration and weight loss tests of polymeric inserts based on methyl hemiester of the Peg1VE/AnM copolymer and containing HSA + IFNₐₗₚₕₐ | | |
|---|---|---|
| Time hours | Weight loss^{a)} % | Hydration^{a)} % |
| 0.5 | 9.0 | 490 |
| 1 | 13.9 | 1850 |
| 3 | 14.8 | 3220 |
| 18 | 15.4 | 3150 |
| 24 | - | 3180 |
| 48 | 17.4 | 3275 |
| 120 | 28.6 | 3240 |

| | | |
|---|---|---|
| a) Average percentage values calculated over three inserts | | |

### EXAMPLES OF "IN VITRO" RELEASE TESTS OF THE DRUG

### Example 18

Three polymeric inserts based on methyl hemiester of the PegIVE/Anm copolymer and containing HSA + ¹²⁵I-IFN_{gamma} of 20 mg average weight each, corresponding to 8.5x10⁻² »ci, were put into a rotating bucket and dipped into 40 ml of a 1.33 mM phosphate buffer solution at pH 7.4 kept at 37°C ± 0.1°C. At suitable intervals, 2 ml of the solution were drawn out, substituting them with the same amount of fresh buffer solution.

**Table 3**

| Release of interferon from inserts based on methyl hemiester of the Peg1VE/AnM copolymer and containing HSA and ¹²⁵I-IFN_{gamma} | | |
|---|---|---|
| Time (hours) | Cpm | R^{a)} (%) |
| 0.25 | 670 | 2.7 |
| 0.75 | 1164 | 4.8 |
| 1.5 | 1241 | 5.1 |
| 3 | 1395 | 5.7 |
| 6 | 1496 | 6.1 |
| 12 | 2054 | 8.4 |
| 21 | 2859 | 11.7 |

| | | |
|---|---|---|
| a) Ratio between the cpm given by the insert solutions and the cpm of a reference dry insert | | |

The amount of ¹²⁵I-IFN_{gamma} released was determined (cf. Table 3) on the basis of radioactivity as counts per minute (cpm) of the solution drawn, with respect to the radioactivity as cpm of a reference dry insert (Figure 3).

## Claims

1. A hydrogel matrix for sustained release of compounds having pharmacological activity, said matrix being characterized in that it comprises a hydrocarbon carbon chain synthetic polymer having the formula: 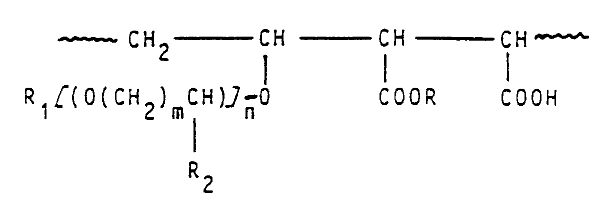 wherein R = (C₁-C₁₂)-alkyl; R₁ = (C₁-C₄)-alkyl; R₂ = H (m = 1,3; n = 1-12) or CH₃ (m = 1; n = 1-12); a protein component and a pharmacologically active principle.

2. A hydrogel matrix for sustained release of compounds which are endowed with pharmacological activity accordingo to claim 1, said matrix being characterized in that said synthetic polymer is derived by polymerization of maleic anhydride with a monomethoxyoligoethylene glycol vinyl ether and successive hemiesterification by means of alcohols.

3. A hydrogel matrix for sustained release of compounds which are endowed with pharmacological activity according to claim 2, said matriw being characterized in that said monoalkyloxyoligoethylene glycol vinyl ether is included in the group of 2-methoxyethylvinyl ether, monomethoxytriethylene glycol vinyl ether and monomethoxytetraethylene glycol vinyl ether.

4. A hydrogel matrix for sustained release of compounds which are endowed with pharmacological activity according to anyone of the preceding claims, said matrix being characterized in that said protein component is an albumin.

5. A hydrogel matrix for sustained release of compounds which are endowed with pharmacologial activity according to claim 4, characterized in that said albumin is human seric albumin (HSA).

6. A hydrogel matrix for sustained release of compounds which are endowed with pharmacological activity accordin to any one of the preceding claims, characterized in that said pharmacologically active principle comprises a protein or a polypeptide.

7. A hydrogel matrix for sustained release of compounds which are endowed with pharmacological activity according to claim 6, said matrix being characterized in that said protein or polypeptide comprises an interferon.

8. A hydrogel matrix for sustained release of pharmacological active compounds according to claim 7, said matrix being characterized in that said interferon is beta-interferon.

9. A hydrogel matrix for sustained release of compounds which are endowed with pharmacological activity according to any one of the preceding claims, characterized in that said sustained release occurs through the transcutaneous, transdermal or phthalmic route.

10. Ophthalmic inserts obtained by means of the hydrogel matrix for sustained release of compounds which are endowed with pharamcological activity according to claim 9.

11. A process for the preparation of hydrogel matrices for controlled release of compounds which are endowed with pharmacological activity according to any one of the preceding claims, said process being characterized in that it comprises the step of:
- preparing by means of polymerization techniques said synthetic polymers, and converting the same into hemiesters by reaction with alcohols;
- adding said protein comoonent to a solution of said synthetic polymer;
- controlled evaporation.

12. A process for the preparation of hydrogel matrices for sustained release of compounds which are endowed with pharmacological activity according to claim 11, said process being characeterized in that said polymerization occurs between maleic anhydride and a monomethoxyoligoethylene glycol vinyl ether.

13. A process for the preparation of hydrogel matrices for sustained release of compounds endowed with pharmacological activity according to claim 12, said process being characterized in that said monomethoxyoligoethylene glycol vinyl ether is included in the group of: 2-methoxyethyl vinyl ether, mnomthoxytriethylene glycol vinyl ether and monomethoxytetraethylene glycol vinyl ether.

14. A process for the preparation of hydrogel matrices for sustained release of compounds which are endowed with pharmacological activity according to any one of the preceding claims 11-13, said process being characterized in that said hemiesterification is carried out by reaction with alcohols.

15. A process for the preparation of hydrogel matrices for sustained release of compounds which are endowed with pharmacological activity according to claim 14, said process being characterized in that said alcohols are included in the group consisting of methanol, n-butanol and lauryl alcohol.

16. A process for the preparation of hydrogel matrices for sustained release of compounda which are endowed with pharamcological activity according to any one of the preceding claims 11-15, said process being characterized in that said protein component is added to a solution od said synthetic polymer in organic solvents, or in water-organic or water-based solvents, wherein said polymer is present at 1 % and 50 % by weight and wherein the weight ratio of the protein component to the synthetic polymer is between 0.01 and 1.

17. A process for the preparation of hydrogel matrices for sustained release of compounds which are endowed with pharmacological activity according to claim 16, said process being characterized in that said weight ratio of the protein component to the synthetic polymer is between 0.1 and 0.5.

18. A process for the preparation of hydrogel matrices for sustained release of compounds which are endowed with pharmacological activity according to any one of the preceding claims 11-17, said process being characterized in that said controlled evaporation occurs at a temperature between -70°C and 0°C, and next between 0°C and 50°C.

19. A process for the preparation of hydrogel matrices for sustained release of compounds which are endowed with pharmacological activity according to any one of the preceding claims 11-18, said process being characterized in that said matrices form films of thickness between 10 and 500 »m.

20. A process for the preparation of hydrogel matrices for sustained release of compounds which are endowed with pharmacological activity according to any one of the preceding claims 11-19, said process being characterized in that a cross-linking agent is added in amounts between 0.2 and 20 % by weight.

21. A process for the preparation of hydrogel matrices for sustained release of compouds which are endowed with pharmacological activity according to claim 20, said process being characterized in that said cross-linking agent is of divinyl, epoxide, isocyanic or amino-nature.

## Patentansprüche

1. Hydrogel- Matrix fuer verzoegerte Freisetzung von pharmakologische Wirkung aufweisenden Verbindungen, dadurch gekennzeichnet, dass sie ein synthetisches Polymer mit einer Kohlenwasserstoff- Kette der Formel 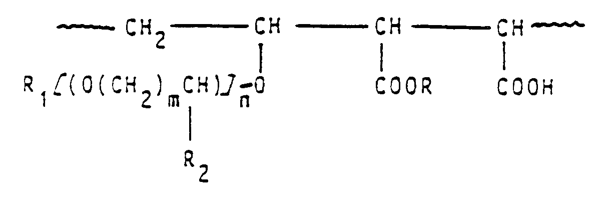 aufweist, worin R = (C₁-C₁₂)-Alkyl; R₁ = (C₁-C₄)-Alkyl; R₂ = H (m = 1,3; n = 1-12) oder CH₃ (m = 1,3; n = 1-12); ein Protein-Bestandteil und ein pharmakologisch aktiver Wirkstoff.

2. Hydrogel-Matrix fuer verzoegerte Freisetzung von pharmakologische Wirkung aufweisenden Verbindungen nach Anspruch 1, dadurch gekennzeichnet, dass das vorgenannte synthetische Polymer durch Polymerisation von Maleinsaeure-Anhydrid mit einem Monomethoxyoligoaethylen-Glycovinyl-Aether und nachherige Hemiveresterung durch Alkhole erhalten wird.

3. Hydrogel-Matrix fuer verzoegerte Freisetzung von pharmakologische Wirkung aufweisenden Verbindungen nach Anspruch 2, dadurch gekennzeichnet, dass das vorgenannte Monomethoxyoligoaethylen-Glycolvinyl-Aether zur Gruppe von 2-Methoxy-aethylvinyl-Aether, Monomethoxytriaethylen-Glycolvinyl-Aether und Monomethoxytetraaethylen-Glycovinyl-Aether gehoert.

4. Hydrogel-Matrix fuer verzoegerte Freisetzung von pharmakologische Wirkung aufweisenden Verbindungen nach je einem der vorhergehenden Ansprueche, dadurch gekennzeichnet, dass der vorgenannte Protein-Bestandteil ein Albumin ist.

5. Hydrogel-Matrix fuer verzoegerte Freisetzung von pharmakologische Wirkung aufweisenden Verbindungen nach Anspruch 4, dadurch gekennzeichnet, dass das vorgenannte Albumin ein menschliches Serumalbumin (HSA) ist.

6. Hydrogel-Matrix fuer verzoegerte Freisetzung von pharmakologische Wirkung aufweisenden Verbindungen nach je einem der vorhergehenden Ansprueche, dadurch gekennzeichnet, dass das vorgenannte pharmakologisch aktiver Wirkstoff ein Protein oder ein Polypeptid enthaelt.

7. Hydrogel-Matrix fuer verzoegerte Freisetzung von pharmakologische Wirkung aufweisenden Verbindungen nach Anspruch 6, dadurch gekennzeichnet, dass das vorgenannte Protein oder Polypetid ein Interferon enthaelt.

8. Hydrogel-Matrix fuer verzoegerte Freisetzung von pharmakologische Wirkung aufweisenden Verbindungen nach Anspruch 7, dadurch gekennzeichnet, dass das vorgenannte Interferon ein Beta-Interferon ist.

9. Hydrogel-Matrix fuer verzoegerte Freisetzung von pharmakologische Wirkung aufweisenden Verbindungen nach je einem der vorhergehenden Ansprueche, dadurch gekennzeichnet, dass die vorgenannte verzoegerte Freisetzung durch transkutane, transdermatische oder ophtalmische Wege erfolgt.

10. Ophtalmische Einsaetze erhalten durch die Hydrogel-Matrix fuer verzoegerte Freisetzung von pharmakologische Wirkung aufweisenden Verbindungen nach Anspruch 9.

11. Verfahhren zur Vorbereitung von Hydrogel- Matrizes fuer verzoegerte Freisetzung von pharmakologische Wirkung aufweisenden Verbindungen nach je einem der vorhergehenden Ansprueche, dadurch gekennzeichnet, dass es die folgenden Verfahrensschritte aufweist:
- Vorbereitung durch technische Polymerisation der vorgenannten Kunstpolymere und Umsetzung derselben in Hemiester durch Reaktion mit Alkoholen;
- Zugabe de vorgenannten Protein-Bestandteils zu einer Loesung des vorgenannten Kunstpolymers;
- gesteuerte Verdampfung.

12. Verfahhren zur Vorbereitung von Hydrogel- Matrizes fuer verzoegerte Freisetzung von pharmakologische Wirkung aufweisenden Verbindungen nach Anspruch 11, dadurch gekennzeichnet, dass die vorgenannte Polymerisation zwischen Maleinsaeureanhydrid und einem Monomethoxyoligoaethylen- Glycolvinyl- Aether stattfindet.

13. Verfahhren zur Vorbereitung von Hydrogel- Matrizes fuer verzoegerte Freisetzung von pharmakologische Wirkung aufweisenden Verbindungen nach Anspruch 12, dadurch gekennzeichnet, dass der vorgenannte Monomethoxyoligoaethylen-Glycolvinyl-Aether zur Gruppe von: 2-Methoxyaethyl-Vinyl-Aether, Monomethoxy-triaethylen-Glycolvinyl-Aether und Monomethoxytetraaethylen-Glycolvinil-Aether gehoert.

14. Verfahhren zur Vorbereitung von Hydrogel- Matrizes fuer verzoegerte Freisetzung von pharmakologische Wirkung aufweisenden Verbindungen nach den Anspruechen 11 bis 13, dadurch gekennzeichnet, dass die vorgenannte Hemiveraesterung durch Reaktion mit Alkoholen durchgefuehrt wird.

15. Verfahhren zur Vorbereitung von Hydrogel- Matrizes fuer verzoegerte Freisetzung von pharmakologische Wirkung aufweisenden Verbindungen nach Anspruch 14, dadurch gekennzeichnet, dass die vorgenannten Alkohole zur aus Methanol, n-Butanol and Lauryl-Alkohol bestehenden Gruppe gehoeren.

16. Verfahhren zur Vorbereitung von Hydrogel- Matrizes fuer verzoegerte Freisetzung von pharmakologische Wirkung aufweisenden Verbindungen nach den Anspruchen 11-15 dadurch gekennzeichnet der vorgenannte Proteinbestandteil zu einer Loesung des vorgenannten Kunstpolymers in organischen Loesungsmitteln oder in wasserorganischen oder Wasserloesungen zugegeben wird, wobei das vorgenannte Polymer in einem Gewicht-Anteil von 1% bis 50% enthalten ist und das Gewicht-Verhaeltnis zwischen dem Proteinbestandteil und dem Kunstpolymer zwischen 0.01 und 1 vorliegt.

17. Verfahhren zur Vorbereitung von Hydrogel- Matrizes fuer verzoegerte Freisetzung von pharmakologische Wirkung aufweisenden Verbindungen nach Anspruch 16, dadurch gekennzeichnet dass das Gewicht-Verhaeltnis des Proteinbestandteils zum Kunstpolymer von 0.1 bis 0.5 ist.

18. Verfahhren zur Vorbereitung von Hydrogel- Matrizes fuer verzoegerte Freisetzung von pharmakologische Wirkung aufweisenden Verbindungen nach den Anspruchen 11 bis 17, dadurch gekennzeichnet, dass die vorgenannte gesteuerte Verdampfung bei einer Temperatur zwischen -70°C und 0°C und dann zwischen 0°C und 50°C durchgefuehrt wird.

19. Verfahhren zur Vorbereitung von Hydrogel- Matrizes fuer verzoegerte Freisetzung von pharmakologische Wirkung aufweisenden Verbindungen nach den Anspruchen 11 bis 18, dadurch gekennzeichnet, dass die vorgenannten Matrizes in Filmform mit einer Staerke zwischen 10 und 500 »m vorliegen.

20. Verfahhren zur Vorbereitung von Hydrogel- Matrizes fuer verzoegerte Freisetzung von pharmakologische Wirkung aufweisenden Verbindungen nach den Anspruchen 11 bis 19, dadurch gekennzeichnet ein Vernetzungsmittel in einem Gewicht-Anteil zwischen 0,2 und 20% zugegeben wird.

21. Verfahhren zur Vorbereitung von Hydrogel- Matrizes fuer verzoegerte Freisetzung von pharmakologische Wirkung aufweisenden Verbindungen nach Anspruch 20, dadurch gekennzeichnet, dass das vorgenannte Vernetzungsmittel der divinylischen, epoxydischen, isozyanische oder Amino-Art ist.

## Revendications

1. Matrice à hydrogel pour la libération soutenue des compositions pharmacologiquement actives, caractérisée en ce qu'elle comprend un polymère synthétique à chaine de hydrocarbures de la formule: 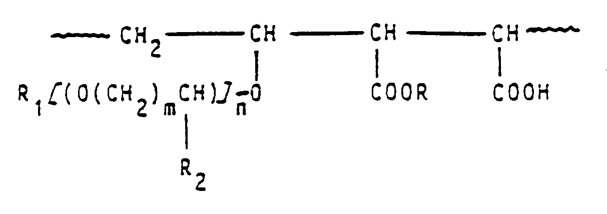 dans laquelle R = (C₁-C₁₂)-alcoyle; R₁ = (C₁-C₄)-alcoyle; R₂ = H(m = 1,3; n = 1-12) ou CH₃ (m = 1; n = 1-12); un composant protéique et un agent pharmacologiquement actif.

2. Matrice à hydrogel pour la libération soutenue des compositions pharmacologiquement actives selon la revendication 1, caractérisée en ce que ledit polymère synthétique est derivé par polymèrisation d'anhydride malèique avec un monomèthoxyoligoéthylène glicol vinyl éther et par hémiestérification successive au moyen des alcools.

3. Matrice à hydrogel pour la libération soutenue des compositions pharmacologiquement actives selon la revendication 2, caractérisée en ce que ledit monoèthoxyoligoéthylène glicol vinyl éther est compris dans le groupe consistant de 2-mèthoxyéthylvinyl éther, monomèthoxytriéthylène glycol vinyl éther et monomèthoxytetraéthylène glycol vinyl éther.

4. Matrice à hydrogel pour la libération soutenue des compositions pharmacologiquement actives selon une quelconque des revendications précédentes, caractérisée en ce que ledit composant protéique est une albumine.

5. Matrice à hydrogel pour la libération soutenue des compositions pharmacologiquement actives selon la revendication 4, caractérisée en ce que ladite albumine est une albumine sérique humaine.

6. Matrice à hydrogel pour la libération soutenue des compositions pharmacologiquement actives selon une quelconque des revendications précédentes, caractérisée en ce que ledit agent pharmacologiquement actif comprend une protéine ou un polypeptide.

7. Matrice à hydrogel pour la libération soutenue des compositions pharmacologiquement actives selon la revendication 6, caractérisée en ce que ladite protéine ou ledit polypeptide comprend un interferon.

8. Matrice à hydrogel pour la libération soutenue des compositions pharmacologiquement actives selon la revendication 7, caractérisée en ce que ledit interferon est un beta-interferon.

9. Matrice à hydrogel pour la libération soutenue des compositions pharmacologiquement actives selon une quelconque des revendications précédentes, caractérisée en ce que ladite libération soutenue a lieu atravers la route transcutanée, transdermale ou ophtalmique.

10. Inserts ophtalmiques obtenus au moyens de matrices à hydrogel pour la libération soutenne des compositions pharmacologiquement actives selon la revendication 9.

11. Procédé pour la préparation des matrices à hydrogel pour la libération controllée des compositions pharmacologiquement actives selon une quelconque des revendications précédentes, caractérisé en ce qu'il comprend les phases de:
- préparation par polymèrisation technique desdits polymères synthétiques et leur conversion en hémiesters par réaction avec des alcools;
- addition dudit composant protéique à une solution dudit polymère synthetique;
- évaporation controlée.

12. Procédé pour la préparation des matrices à hydrogel pour la libération controllée des compositions pharmacologiquement actives selon la revendication 11, caractérisé en ce que ladite polymèrisation a lieu entre l'anhydride malèique et un monomèthoxyoligoéthylène glycol vinyl éther.

13. Procédé pour la préparation des matrices à hydrogel pour la libération controllée des compositions pharmacologiquement actives selon la revendication 12, caractérisé en ce que ledite monomèthoxyoligoéthylène glycol vinyl éther est compris dans le groupe de: 2-mèthoxyéthyl vinyl éther, monomèthoxytiéthylène glycol vinyl éther et monomèthoxytetraéthylène glycol vinyl éther.

14. Procédé pour la préparation des matrices à hydrogel pour la libération controllée des compositions pharmacologiquement actives selon une quelconque des revendications précédentes 11-13, caractérisé en ce que ladite hémiesterification est réalisée par réaction avec des alcools.

15. Procédé pour la préparation des matrices à hydrogel pour la libération controllée des compositions pharmacologiquement actives selon la revendication 14, caractérisée en ce que lesdits alcools son inclus dans le groupe consistant de méthanol, n-butanol et lauryl alcool.

16. Procédé pour la préparation des matrices à hydrogel pour la libération controllée des compositions pharmacologiquement actives selon une quelconque des revendications 11-15, caractérisé en ce que ledite composant protéique est ajouté à une solution dudit polymére synthetique dans solvants organiques ou dans solvants organique d'eau ou à base d'eau, dans la quelle ledit polymére est présent entre 1% et 50% en poids et dans laquelle le rapport du composant protéique au polymére synthetique est compris entre 0,01 et 1.

17. Procédé pour la préparation des matrices à hydrogel pour la libération controllée des compositions pharmacologiquement actives selon la revendication 16, caractérisé en ce que ledit rapport du composant protéique au polymére synthetique est compris entre 0,1 et 0,5.

18. Procédé pour la préparation des matrices à hydrogel pour la libération controllée des compositions pharmacologiquement actives selon une quelconque des revendications 11-17, caractérisé en ce que ladite évaporation controllée a lieu à une tempèrature entre - 70°C et aprés entre 0°C et 50°C.

19. Procédé pour la préparation des matrices à hydrogel pour la libération controllée des compositions pharmacologiquement actives selon une quelconque des revendications 11-18, caractérisé en ce que lesdites matrices torment des films d'épaisseur entre 10 et 500 »m.

20. Procédé pour la préparation des matrices à hydrogel pour la libération controllée des compositions pharmacologiquement actives selon une quelconque des revendications 11-19, caractérisé en ce que un agent de rétification est ajouté en quantité entre 0,2 et 20% en poids.

21. Procédé pour la préparation des matrices à hydrogel pour la libération controllée des compositions pharmacologiquement actives selon la revendication 20, caractérisé en ce que ledit agent de rétification est de nature divinylique, époxyde, isocyanique ou de ammino-nature.
